# EUROPEAN PATENT SPECIFICATION

(11) **EP 2 090 216 B1**
(45) Date of publication and mention of the grant of the patent: **27.08.2014**
(21) Application number: 09001974.6
(22) Date of filing: 12.02.2009
(51) Int. Cl.: A61B 1/00

(54) **Processor for endoscope and endoscope system**
Prozessor für Endoskope und Endoskopsystem
Processeur pour endoscope et système d'endoscope

(30) Priority: 14.02.2008 JP 2008032729
(43) Date of publication of application: 19.08.2009
(73) Proprietor: FUJIFILM Corporation, Minato-ku Tokyo (JP)
(72) Inventor: Higuchi, Mitsuru, Saitama-shi, Saitama (JP)
(74) Representative: Höhfeld, Jochen

(56) References cited:
- EP-A- 0 018 125
- JP-A- 2000 092 478
- JP-A- 2005 198 844
- US-A- 4 667 230
- US-A- 5 554 893
- US-A1- 2004 171 913

## Description

### FIELD OF THE INVENTION

The present invention relates to a processor for an endoscope, according to the preamble of claim 1.

### BACKGROUND OF THE INVENTION

An endoscope system is constituted of an endoscope (scope) and a processor. The endoscope is used for taking images inside a body cavity. The endoscope is detachably connected to the processor via a connector. The processor performs image processing to image data transmitted from the endoscope, and outputs the image data to a display. The endoscope has an image sensor such as a CCD at a distal tip of an insertion section that is inserted in a body cavity. The endoscope increases in size and weight when a power supply and a drive circuit for the CCD are provided in the endoscope, which makes the endoscope difficult to handle. For this reason, it is preferred to provide the power supply and the drive circuits in the processor so as to supply the power and output the drive signals from the processor to the endoscope.

To remove the endoscope from the processor, it is necessary to stop the CCD and the like in advance, and check that the endoscope is ready for removal. However, since a doctor delegates a task to stop the CCD, and is extremely busy in a medical site, the CCD is often left activated and the endoscope is removed while the CCD is still driven. In such cases, when a line for the power supply is disconnected before a line for the drive signals is disconnected, the drive signal may cause unexpected currents in a circuit of the CCD, and may damage the CCD.

To solve the above problem, in Japanese Patent Laid-Open Publication No. 2000-092478, a plug connector is provided with pins shorter than the rest of the pins of the plug connector. Such shorter pins are used for controlling power supply. The power is supplied and control signals are input to the endoscope only when the shorter pins are conducted. When the endoscope is removed, the shorter pins are disconnected first, which stops the power supply and input of the control signals to the endoscope. As a result, the CCD is protected from damage even if the endoscope is removed while the CCD is driven.

However, a plug connector having pins shorter or longer than the rest of the pins is not commercially available. Therefore, in order to adopt the configuration described in Japanese Patent Laid-Open Publication No. 2000-092478, it is necessary to customize a plug connector, incurring a substantial amount of cost. In addition, Japanese Patent Laid-Open Publication No. 2000-092478 does not provide a solution to prevent damage of the conventional endoscope with the ordinary plug connector having the pins of the same length.

US-A-5 554 893 discloses a processor according to the preamble of claim 1. Specifically, the processor connector and the endoscope connector have a structure including a connection ring having a female thread adapted to be threadedly engaged with a male thread provided at the processor connector side. When the connection ring is threaded onto the male thread, the ring is moved axially and engages an operating portion of a switch which connects a power source circuit to a control circuit, whereupon the control circuit supplies a constant voltage to the image sensor prior to supplying a driving signal to the image sensor. When the processor connector and the endoscope connector are disconnected, the control circuit detects the off-state of said switch which is opened by the operating portion cooperating with the connection ring. Then, the drive signal supply is terminated and then the constant voltage supply is terminated.

EP-A-0 018 125 discloses an endoscope having a connector adapted to be coupled to a camera apparatus, wherein the camera apparatus includes a film winding motor and the like. In order to supply the film winding motor and further electric and electronic parts of the camera with power, in one embodiment, a first terminal of the camera connected to a battery and a second terminal of the camera connected to the electrical and electronic parts are short-circuited by a ring shape metal member included in the coupling part of the endoscope.

### SUMMARY OF THE INVENTION

An object of the present invention is to provide a processor for an endoscope and an endoscope system that prevents damage to a CCD when an endoscope is removed from the processor while the CCD is driven, without modifying a connector.

In order to achieve the above objects and other objects, a processor of the present invention includes the features of claim 1.

It is preferred that each of the processor connector and the endoscope connector comprises a flat type connector having the contacts linearly aligned in a direction substantially orthogonal to an insertion and removal direction of the endoscope connector. The detecting section is constituted of a first detector and a second detector, and the first detector is provided at a side end portion of the processor connector, and the second detector is provided at the other side end portion of the processor connector.

It is preferred that the first detector is a first contact, and the second detector is a second contact. The first contact is provided at the side end portion of the processor connector and the second contact is provided at the other side end portion of the processor connector. The detection section detects start of removal of the endoscope connector when conduction between at least one of the first and second contacts and corresponding contact of the endoscope connector ceases.

It is preferred that the detection section includes a third detector that detects start of removal of the endoscope connector by sensing a mechanical displacement of the endoscope connector.

It is preferred that the third detector includes an arm, a biasing member, and a sensor. The arm is movable between a first position in which the arm contacts with the processor connector, and a second position in which the arm is pushed away from the first position and comes in contact with the endoscope connector connected to the processor connector. The biasing member pushes the arm against the first position. The sensor senses movement of the arm from the first position.

It is preferred that the third detector is located close to a center portion of the processor connector.

It is preferred that start of removal of the endoscope connector is detected by the third detector before being detected by the first and the second detectors.

It is preferred that the contacts used for the power supply are disposed close to a center portion of each of the endoscope connector and processor connector.

An endoscope system of the present invention includes an endoscope and a processor according to the invention.

According to the present invention, the output of the drive signals and power supply are stopped in this order in response to detection of the start of removal of the endoscope connector. Thereby, the image sensor is prevented from damage when the endoscope is removed from the processor in a state that the image sensor is driven. In addition, since it is not necessary to modify the existing connectors of the endoscope and the processor, additional cost to develop new connectors for preventing the damage of the image sensor is not necessary. Thus, the CCD of the existing endoscopes is also prevented from damage.

### BRIEF DESCRIPTION OF THE DRAWINGS

Figure 1 is a perspective view of an endoscope system;
Figure 2 is a partial perspective view of a first control connector;
Figure 3 is a partial perspective view of a second control connector;
Figure 4 is an explanatory view showing function of each contact;
Figure 5 is a block diagram of an electronic endoscope and a processor;
Figure 6 is an explanatory view of the first control connector pulled out in a tilted direction;
Figure 7 is a flow chart of steps of inspection using the endoscope system;
Figure 8 is an explanatory view of an embodiment with a detection mechanism;
Figure 9 is an exploded perspective view of the detection mechanism; and
Figures 10A and 10B are explanatory views of an arm that swingably moves between a normal position and a connection detecting position.

### DESCRIPTION OF THE PREFERRED EMBODIMENTS

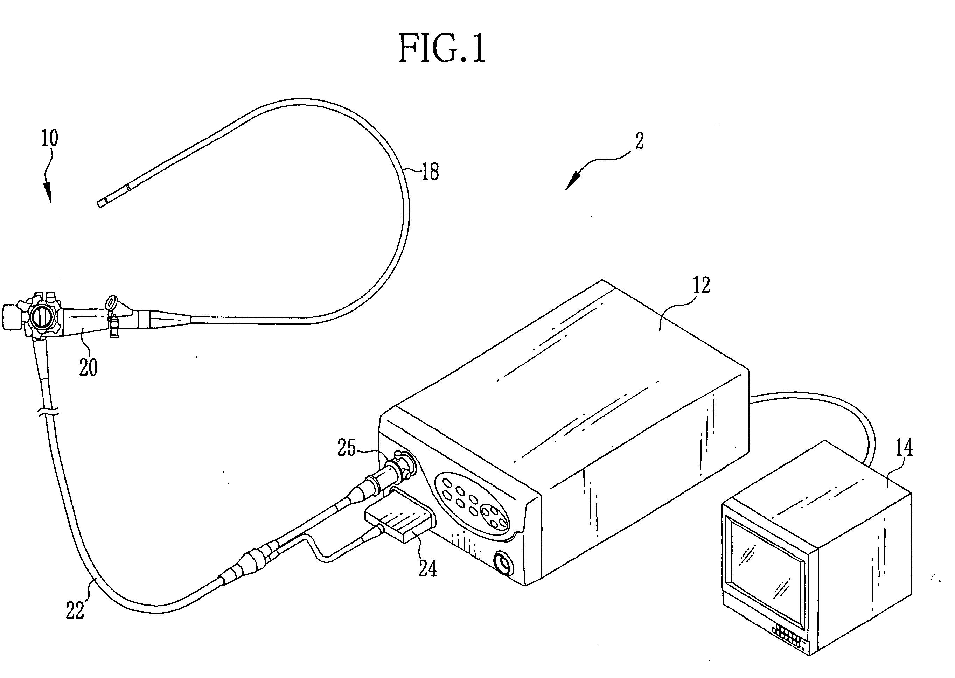

In Fig.1, an endoscope system 2 includes an electronic endoscope (hereinafter may simply be referred to as endoscope) 10, a processor 12, and a monitor (display device) 14. Images of a body cavity of a patient are taken using the endoscope 10. The processor 12 generates endoscopic images. The monitor 14 displays the endoscopic images. The endoscope 10 has an insertion section 18, an operating section 20, and a universal cord 22. The insertion section 18 is inserted into the body cavity of the patient. The operating section 20 is connected to a base end portion of the insertion section 18, and hand-operated. The universal cord 22 extends from the operating section 20. The processor 12 is integrated with a light source for illuminating the body cavity. At an end of the universal cord 22 on the opposite side of the operating section 20 are provided a first control connector (endoscope connector) 24 and a first light source connector 25. The first control connector 24 is used for transmitting power and various control signals. The first light source connector 25 is used for taking in illumination light from the light source. The endoscope 10 is detachably connected to the processor 12 via the first control connector 24 and the first light source connector 25.

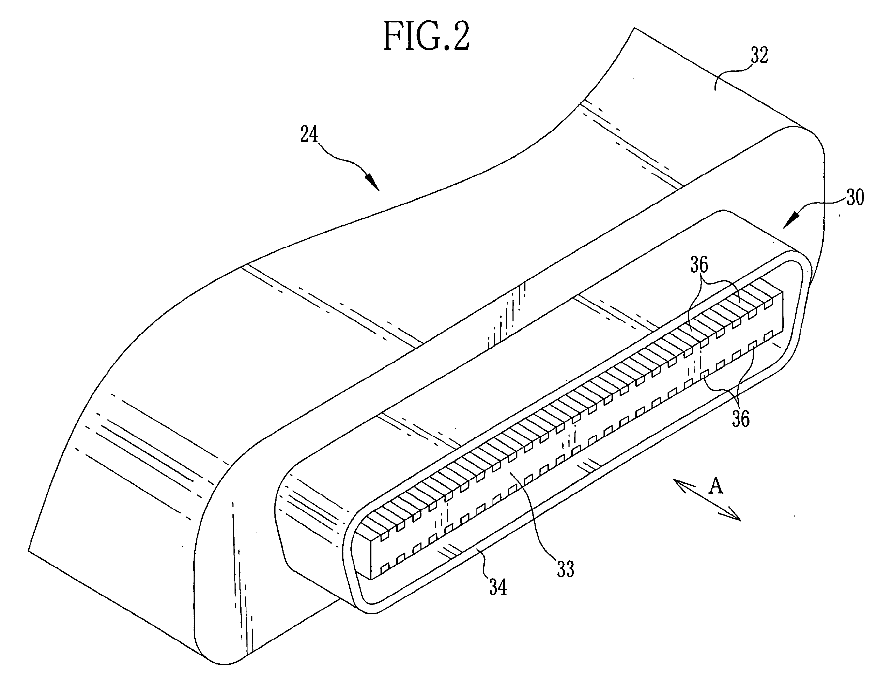

As shown in Fig. 2, the first control connector 24 is constituted of a connect section 30 and a connector housing 32. The first control connector 24 is mechanically and electrically connected to the processor 12 via the connect section 30. The connector housing 32 is an approximate rectangular solid. The connector housing 32 covers and protects a connect portion between the connect section 30 and the universal cord 22. The connect section 30 has an approximately flat fitting plate 33, and a fitting tube 34 surrounding the fitting plate 33. Each of upper and lower surfaces of the fitting plate 33 is provided with 25 contacts 36 arranged at regular intervals. Namely, the first control connector 24 is a so-called Amphenol (registered trademark)-type plug connector, and has a flat structure on which the contacts 36 are linearly aligned in a direction approximately orthogonal to insertion and removal directions indicated by an arrow A (hereinafter referred to as direction A).

The fitting tube 34 is a rectangular tube having a trapezoidal cross-section with a side above the fitting plate 33 longer than a side below the fitting plate 33. Since the fitting tube is formed to have such asymmetrical shape, a position of the first control connecter 24 for connection is uniquely defined. Accordingly, improper connection of the first control connector 24 is prevented.

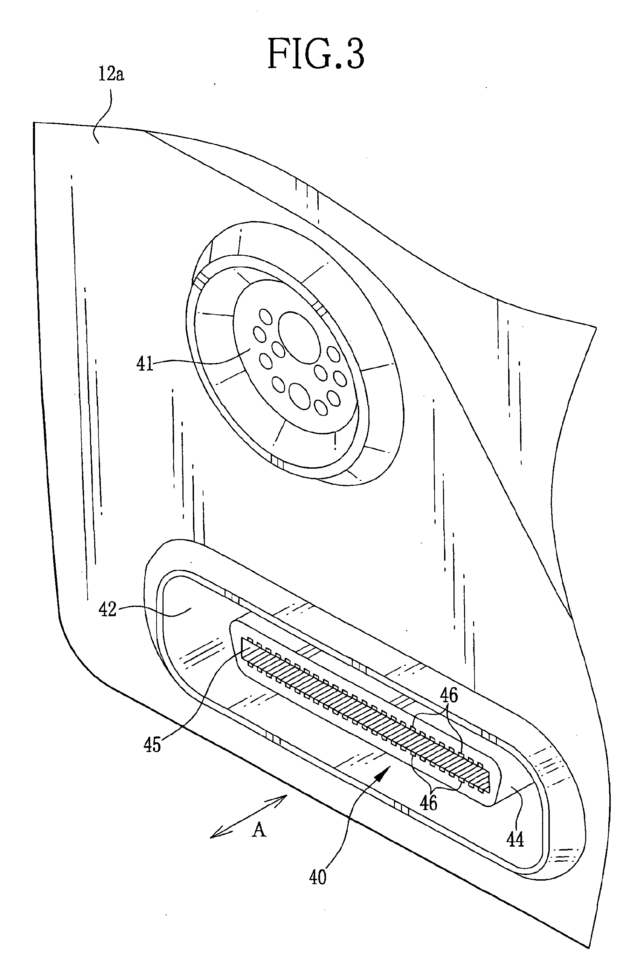

As shown in Fig. 3, a front face 12a of the processor 12 is provided with a second control connector (processor connector) 40 to which the first control connector 24 is connected, and a second light source connector 41 to which the first light source connector 25 is connected. In addition, the front face 12a is provided with a connector retainer 42 that is a hollow portion with a bottom. The second control connector 40 is attached to a bottom surface of the connector retainer 42. The connector retainer 42 has an approximately rectangular opening that matches with the shape of the connector housing 32. The connector housing 32 fits into the connector retainer 42 when the first control connector 24 is connected to the second control connector 40. Thus, the connected first control connector 24 is retained by the connector retainer 42.

The second control connector 40 has a connect section 44 of a rectangular solid shape having an approximately trapezoidal cross-section that fits into the fitting tube 34. A fitting cavity 45 is formed at a position in a connect section 44 where the fitting plate 33 comes into contact when the fitting tube 34 and the connect section 44 are fit. The fitting plate 33 comes into the fitting cavity 45 such that the fitting plate 33 and the fitting cavity 45 are fit. The first and second control connectors 24 and 40 are mechanically connected by the fit between the fitting tube 34 and the connect section 44, and the fit between the fitting plate 33 and the fitting cavity 45.

Upper and lower interior surfaces of the fitting cavity 45 come in contact with the upper and lower surfaces of the fitting plate 33, respectively. Each of the upper and lower interior surfaces of the fitting cavity 45 is provided with 25 contacts 46 aligned at regular intervals. Each contact 46 comes in contact with the corresponding contact 36 when the fitting plate 33 is fit into the fitting cavity 45. The first control connector 24 and the second control connector 40 are electrically connected by contacting the contacts 36 with the contacts 46. The second control connector 40 is a so-called Amphenol-type receptacle connector corresponding to the first control connector 24, and has a flat structure in which the contacts 46 are linearly aligned approximately orthogonal to the direction A.

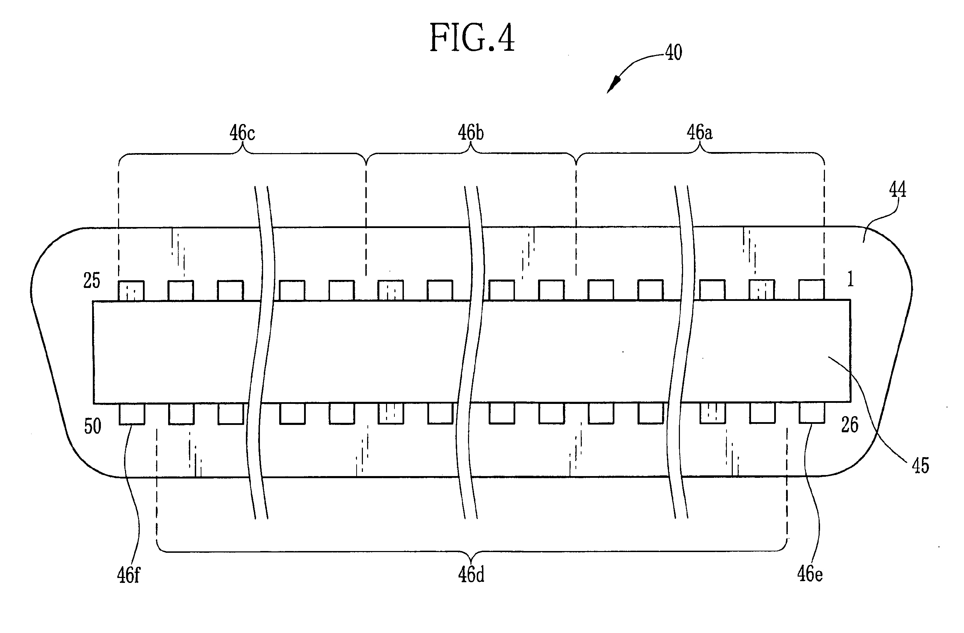

As shown in Fig. 4, when viewed from the front, numbers 1 to 25 are assigned to the contacts 46 from an upper right end to the upper left end of the fitting cavity 45, and numbers 26 to 50 are assigned to the contacts 46 from a lower right end to the lower left end of the fitting cavity 45. Contacts 46a from No. 1 to No. 8 located upper right of the fitting cavity 45 are used for transmitting various control signals between electronic circuits of the endoscope 10 and the processor 12. Contacts 46b from No. 9 to No. 17 located upper center of the fitting cavity 45 are used for supplying the power to the electronic circuit of the endoscope 10. As with the contacts 46a, contacts 46c from No. 18 to No. 25 located upper left of the fitting cavity 45 are used for transmitting various control signals between the electronic circuits of the endoscope 10 and the processor 12.

Contacts 46d from No. 27 to No. 49 located on the lower side of the fitting cavity 45 are used for establishing a common ground between the endoscope 10 and the processor 12. A contact 46e (first detector) of No. 26 located at the lower right end and a contact 46f (second detector) of No. 50 located at the lower left end of the fitting cavity 45 are used for detecting the connection of the endoscope 10. It should be noted that function of each contact 36 of the first control connector 24 is the same as that of the corresponding contact 46 of the second control connector 40.

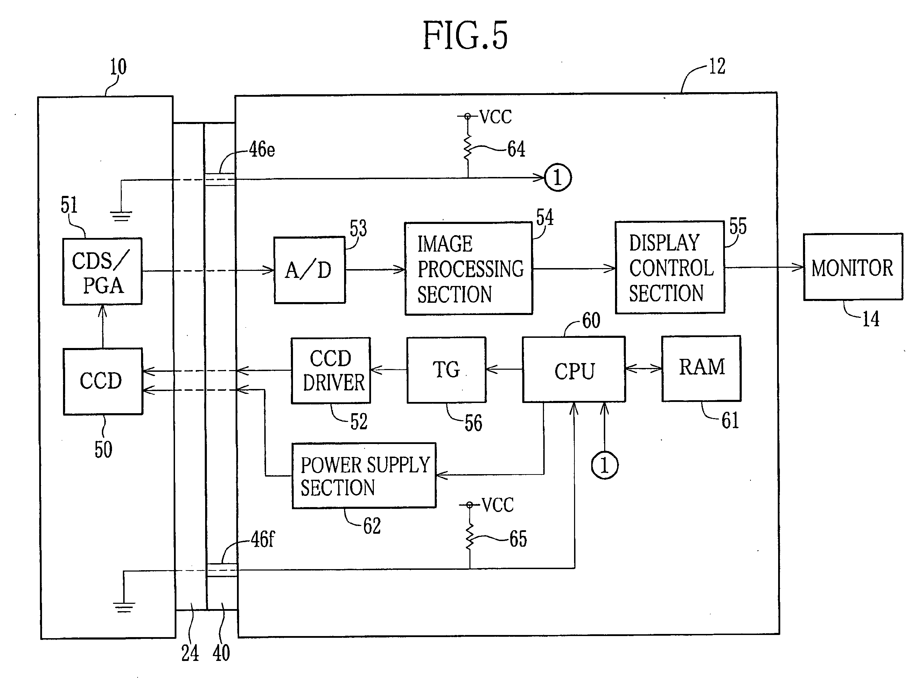

In Fig. 5, the endoscope 10 is provided with a CCD (image sensor) 50 and a correlated double sampling/ programmable gain amplifier (hereinafter abbreviated as CDS/PGA) 51. The CCD 50 captures image light incident from an observation window formed at the distal tip of the insertion section 18. The CDS/PGA 51 removes noise from the image signals output from the CCD 50 and then amplifies the image signals. The CCD 50 is connected to a CCD driver (driver) 52 provided in the processor 12 via the contacts 46a and 46c used for transmitting the control signals. The CDS/PGA 51 is connected to an A/D converter (hereinafter abbreviated as A/D) 53 provided in the processor 12 via the contacts 46a and 46c used for transmitting the control signals.

The A/D 53 converts analog image signals output from the CDS/PGA 51 into digital image data, and outputs the digital image data to an image processing section 54. The image processing section 54 performs various image processing to the digital image data and outputs the processed image data to a display control section 55. The display control section 55 converts the image data output from the image processing section 54 into video signals (component signals, composite signals, or the like) corresponding to a type (display format) of the monitor 14. The video signals are output to the monitor 14. Thereby, endoscopic images of the body cavity of the patient are displayed on the monitor 14.

The CCD driver 52 for driving the CCD 50 is connected to a timing generator (hereinafter abbreviated as TG) 56. The TG 56 is connected to a CPU (controller) 60 that controls each section of the processor 12. Under the control of the CPU 60, the TG 56 inputs timing signals (clock pulses) to the CCD driver 52. The CCD driver 52 inputs the drive signals to the CCD 50 in response to the input timing signals so as to control timing to read accumulated charges, and a shutter speed of an electronic shutter of the CCD 50.

A ROM 61 and a power supply section 62 are connected to the CPU 60. Various programs necessary for controlling the processor 12 are stored in the ROM 61. The power supply section 62 supplies power to the CCD 50. The CPU 60 controls each section of the processor 12 by reading various programs stored in the ROM 61 and sequentially executing the read program. The power supply section 62 is connected to the CCD 50 via the contacts 46b used for supplying the power, and supplies the power to the CCD 50 according to an instruction from the CPU 60.

The contact 46e used for detecting the connection is connected to the CPU 60 and a resistor 64 in parallel. In the same manner, the contact 46f used for detecting the connection is connected to the CPU 60 and a resistor 65 in parallel. A power supply voltage is applied to each of the resistors 64 and 65. The contacts 46e and 46f are connected to ground on the endoscope 10 side when the first and second control connectors 24 and 40 are connected, and the contacts 46e and 46f conduct with the corresponding contacts 36 of the first control connector 24, respectively. The contacts 46e and 46f are connected to ports (not shown) of the CPU 60, respectively. Voltages at these ports of the CPU 60 become high (Hi) when the first control connector 24 and the second control connector 40 are not connected to the ports, and become low (Lo) when the first control connector 24 and the second control connector 40 are connected to the ports.

The CPU 60 detects the connection of the connectors 24 and 40 by monitoring the voltages at the ports to which the contacts 46e and 46f are connected. The CPU 60 detects the connection between the first control connector 24 and the second control connector 40 when the voltages at the both ports become low. The CPU 60 detects start of removal of the first control connector 24 from the second control connector 40 when at least one of the voltages at the ports becomes high.

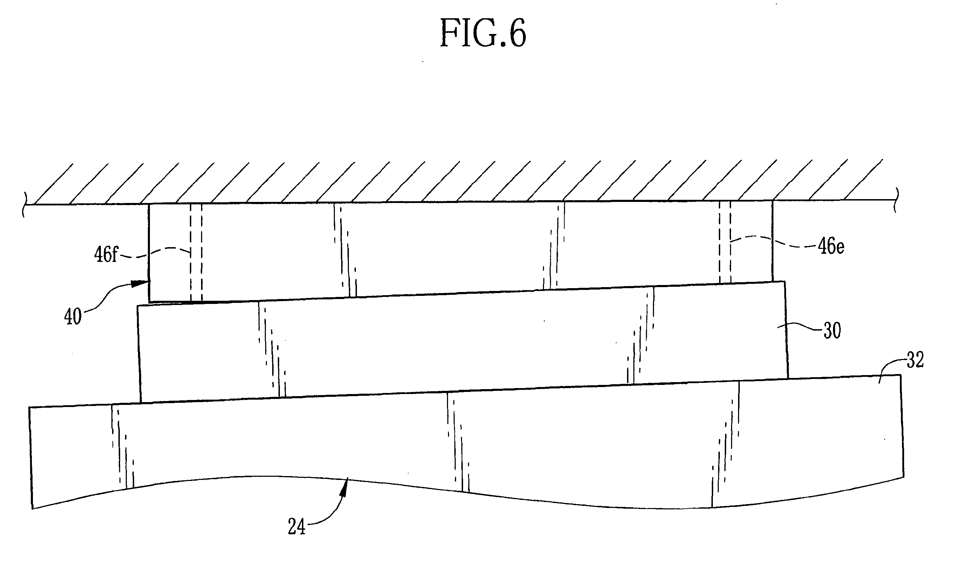

It is rare that the flat-shaped first control connector 24 is straightly pulled out from the second control connector 40 in a direction parallel to the direction A. Usually, the first control connector 24 is slightly tilted and then pulled out as shown in Fig. 6, depending on the conditions of the fitting of the side end portions of the first and the second control connectors 24 and 40, and the force with which the first control connector 24 is applied. Since the contacts 46e and 46f are located at the ends of the lower side of the fitting cavity 45, and the connection of the endoscope 10 is detected by the conduction between the contacts 46e and the corresponding contact 36, and between the contact 46f and the corresponding contact 36, the CPU 60 detects the start of the removal of the first control connector 24 in a state that the contacts 46b used for supplying the power, located upper center of the fitting cavity 45, are electrically connected to the corresponding contacts 36.

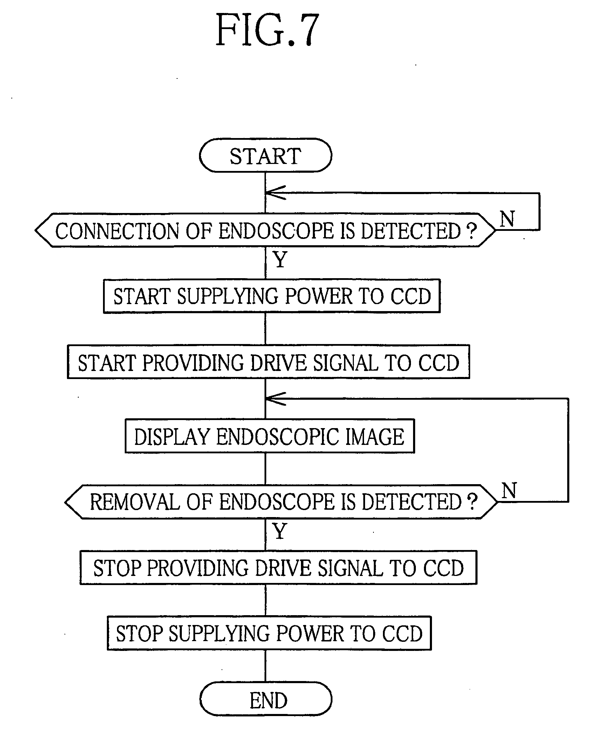

Next, an operation of the endoscope system 2 with the above configuration is described with referring to a flowchart in Fig. 7. To perform an inspection using the endoscope system 2, the first control connector 24 and the first light source connector 25 of the cleaned and sterilized endoscope 10 are inserted to the second control connector 40 and the second light source connector 41, respectively. Thus, the endoscope 10 is connected to the processor 12. When the first control connector 24 is connected to the second control connector 40, the voltages at the ports of the CPU 60 to which the contacts 46e and 46f are connected are changed from high to low. Thus, the connection of the endoscope 10 is detected by the CPU 60.

Upon detecting the connection of the endoscope 10, the CPU 60 instructs the power supply section 62 to supply the power to the CCD 50. The power supply section 62 supplies the power to the CCD 50 accordingly. Thereafter, the CPU 60 starts controlling of the TG 56. Under the control of the CPU 60, the TG 56 inputs the timing signals to the CCD driver 52. Based on the input timing signals, the CCD driver 52 outputs the drive signals to the CCD 50. Thereby, the image signals are output from the CCD 50 and the endoscopic images are displayed on the monitor 14.

When the voltage at one of the ports to which the contacts 46e and 46f are connected becomes high and the CPU 60 detects the start of the removal of the first control connector 24, the CPU 60 stops the drive signals transmitted from the CCD driver 52 to the CCD 50. Thereafter, the CPU 60 instructs the power supply section 62 to stop supplying power to the CCD 50, and the power supply section 62 stops supplying power to the CCD 50 accordingly. Thus, in response to the detection of the start of the removal of the first control connector 24, the drive signals are stopped and the power to the CCD 50 is stopped in this order. As a result, the CCD 50 is prevented from damage caused by disconnecting the endoscope 10 while the CCD 50 is driven.

Since it is not necessary to make changes to the first control connector 24 and the second control connector 40, and commercially available products can be used, no additional costs are necessary to prevent damage of the CCD 50. In addition, since it is not necessary to make changes to the first control connector 24, the CCD of the existing endoscope is prevented from damage.

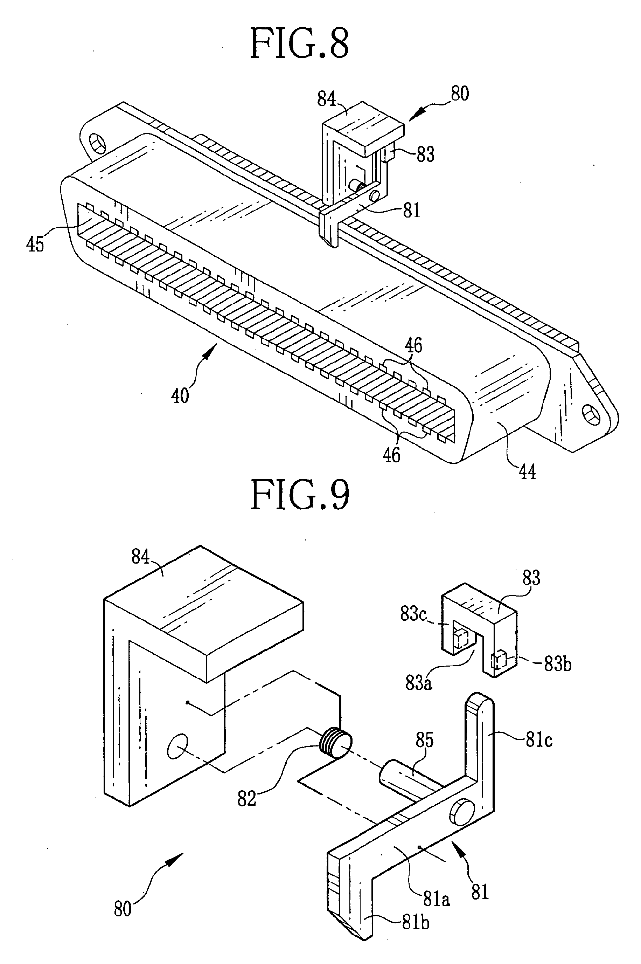

Next, a second embodiment of the present invention is described. A component similar to that of the first embodiment in function or in configuration is designated by the same numeral as the first embodiment, and a description thereof is omitted. As shown in Fig. 8, the processor 12 of this embodiment is provided with a detection mechanism (third detector) 80. The detection mechanism 80 is disposed close to the center of the second control connector 40. The detection mechanism 80 detects attachment and removal of the first control connector 24 by detecting a mechanical displacement of the first control connector 24.

As shown in Fig. 9, the detection mechanism 80 is constituted of an arm 81, a torsion spring (biasing member) 82, a photointerruptor (sensor) 83, and a holder 84. The holder 84 holds the arm 81, the torsion spring 82, and the photointerruptor 83. The holder 84 is attached to a housing or the like of the processor 12.

The arm 81 has a crank-like shape with a long flat plate-like body 81a, a first projection 81b, and a second projection 81c. The first projection 81b extends approximately vertical from an end of the body 81a. The second projection 81c extends approximately vertical from the other end of the body 81a in an opposite direction to the first projection 81b. A shaft 85 is attached to the body 81a. The arm 81 is swingably supported by the holder 84 via the shaft 85.

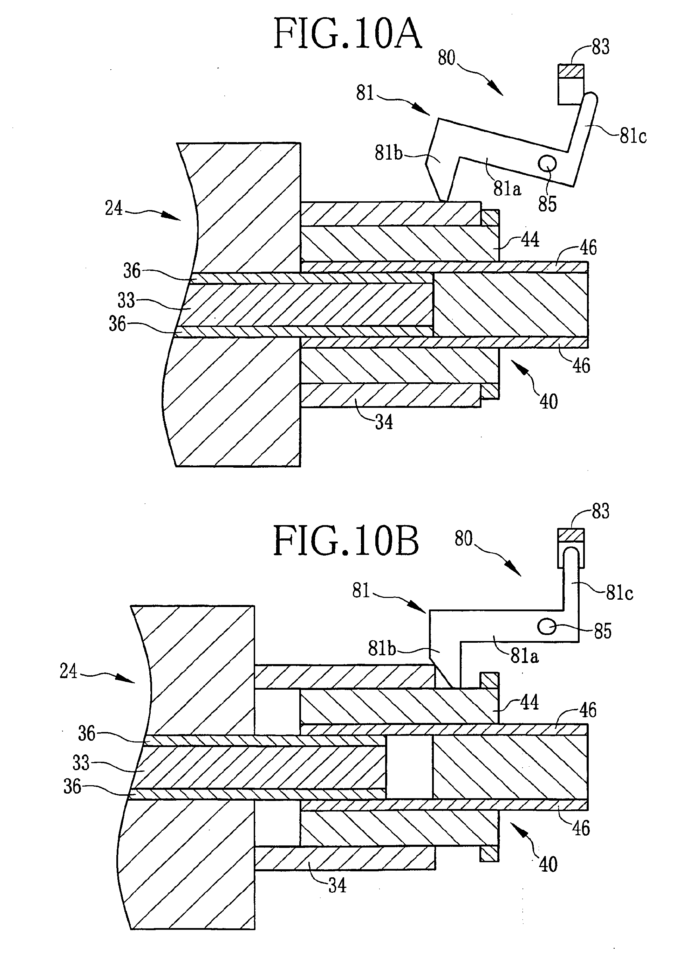

The shaft 85 is inserted through the torsion spring 82. An end of the shaft 85 is connected to the arm 81 while the other end is connected to the holder 84. The torsion spring 82 biases the arm 81 so as to press the arm 81 against the second control connector 40. Thereby, the arm 81 is kept in a normal position (see Fig. 10B) in which the first projection 81b contacts with the second control connector 40 when the first control connector 24 is not connected to the second control connector 40.

The photointerruptor 83 is formed with a detection groove 83a. A light emitting element 83b and a light receiving element 83c face each other across the detection groove 83a. The photointerruptor 83 is a transmission optical sensor that detects interruption of detection light, emitted from the light emitting element 83b, with the use of the light receiving element 83c. The photointerruptor 83 is attached to the holder 84 such that the second projection 81c is inside the detection groove 83a when the arm 81 is in the normal position. The photointerruptor 83 is connected to the CPU 60. When the detection light is detected by the light receiving element 83c, the photointerruptor 83 inputs a high signal (Hi signal) to the CPU 60. When the detection light is not detected by the light receiving element 83c, the photointerruptor 83 inputs a low signal (Lo signal) to the CPU 60.

As shown in Fig. 10A, when the first control connector 24 is connected to the second control connector 40, the fitting tube 34 comes in contact with the first projection 81b. Thereby, the arm 81 moves against the bias of the torsion spring 82, and is pushed upward to a connection detecting position. When the arm 81 is moved to the connection detecting position, the second projection 81c is displaced from the detection groove 83a of the photointerruptor 83 so that the detection light interrupted by the second projection 81c is detected by the light receiving element 83c. Thereby, the voltage at the port to which the photointerruptor 83 is connected is changed from low (Lo) to high (Hi). Thus, the CPU 60 detects the connection of the first control connector 24.

As shown in Fig. 10B, the detection mechanism 80 is configured in a way that the arm 81 returns from the connection detecting section to the normal position before the contacts 36 are completely removed from the contacts 46. Thereby, the CPU 60 detects the start of the removal of the first control connector 24 while the first control connector 24 and the second control connector 40 are electrically connected.

In the above first embodiment, the contacts 46e and 46f located at ends of the lower side of the fitting cavity 45 are used for detecting the start of the removal of the first control connector 24. Therefore, when the first control connector 24 is pulled straight in the direction A, the conduction between the contact 46e and the corresponding contact 36, and the conduction between the contact 46f and the corresponding contact 36 may cease at the same time. As a result, there may be no time to stop the drive signal and the power.

In this embodiment, on the other hand, the detection mechanism 80 disposed close to the center of the second control connector 40 detects the start of the removal of the first control connector 24. Therefore, the drive signal and the power are securely stopped in this order no matter how the first control connector 24 is removed.

It should be noted that the first embodiment and the second embodiment may be performed at the same time. The output of the drive signal and the power supply may be stopped in response to the start of the removal of the first control connector 24 detected by at least one of the detection mechanism 80 and the contacts 46e or 46f. As described above, the detection mechanism 80 is configured in a way that the arm 81 returns from the connection detecting position to the normal position before the contacts 36 are completely removed from the contacts 46. As a result, when the first control connector 24 is pulled out straight, the detection mechanism 80 detects the start of the removal of the first control connector 24 before the conduction between the contacts 46e and the corresponding contact 36, and between the contact 46f and the corresponding contact 36 ceases. On the other hand, when the first control connector 24 is tilted and pulled out, one of the contacts 46e and 46f detects the start of the removal of the first control connector 24 before the detection mechanism 80 detects the removal depending on the degree of the tilt of the first control connector 24. Therefore, with the use of the contacts 46e and 46f and the detection mechanism 80, the start of the removal is more securely detected and a superior level of safety is provided.

In the above embodiments, the contacts 46e, 46f, and the detection mechanism 80 are described as a detecting section. However, the detecting section is not limited to the above. For example, a microswitch, a reflection-type photointerruptor, or any other type of detecting section may be used as long as the start of the removal of the first control connector 24 is detected. In the above embodiments, the detecting section is provided on the processor 12 side. However, the detecting section may be provided on the endoscope 10 side. In the above embodiments, the processor 12 controls to stop the output of the drive signal and the power supply. However, it is not limited to the processor 12. The endoscope 10 may control to stop the output of the drive signal and the power supply.

In the above embodiments, the CCD 50 is used as the image sensor. However, the image sensor is not limited to the above. For example, a CMOS image sensor may be used. In the above embodiments, flat-shaped first and second control connectors 24 and 40 are described. However, the shapes of the first and second control connectors 24 and 40 are not limited to the above. For example, round-shaped connectors may be used. In the above embodiments, the first control connector 24 has 50 contacts 36, and the second control connector 40 has 50 contacts 46. However, the number of the connectors provided to each of the first and the second connectors 24 and 40 is not limited the above.

In the above embodiments, the electronic endoscope 10 is described as an example of the endoscope. However, the endoscope is not limited to the above. The present invention is applicable to, for example, an ultrasonic endoscope. In the above embodiments, the medical endoscope used for inspecting a patient is described. However, the endoscope is not limited to the above. The present invention is applicable to, for example, an industrial endoscope used for inspecting piping or the like. In the above embodiments, the processor 12 integrated with the light source is described. However, the present invention is not limited to the above. The present invention is applicable to a processor with a separate light source.

Various changes and modifications are possible in the present invention and may be understood to be within the present invention.

## Claims

1. A processor (12) for an endoscope (10) comprising:
a processor connector (40) to fit with an endoscope connector (24) provided to said endoscope so as to mechanically and electrically connect said endoscope and said processor;
a power supply section (62) for supplying power via said processor connector and said endoscope connector to an image sensor (50) provided in said endoscope;
a driver (52) for driving said image sensor by providing a drive signal to said image sensor via said processor connector and said endoscope connector;
a detecting section (46e, 46f, 80) for detecting start of removal of said endoscope connector from said processor connector in a state that at least a pair of contacts (46b, 36) for power supply, among a plurality of contacts (46, 36) on said processor connector and said endoscope connector, is kept connected electrically; and
a controller (60) for controlling said driver and said power supply section to stop providing said drive signal first and then stop supplying power in response to detection of start of removal of said endoscope connector,
**characterized in that**
each of said processor connector (40) and said endoscope connector (24) comprises a flat type connector having said contacts linearly aligned in a direction substantially orthogonal to an insertion and removal direction of said endoscope connector, and said detecting section is constituted of a first detector (46e) and a second detector (46f), and said first detector is provided at a side end portion of said processor connector, and said second detector is provided at the other side end portion of said processor connector.

2. The processor of claim 1, wherein said first detector is a first contact (46e) provided at said side end portion, and said second detector is a second contact (46f) provided at said other side end portion, and said detection section detects start of removal of said endoscope connector when conduction between at least one of said first and second contacts and corresponding contact of said endoscope connector ceases.

3. The processor of claim 1, wherein said detection section includes a third detector (80) that detects start of removal of said endoscope connector by sensing a mechanical displacement of said endoscope connector.

4. The processor of claim 3, wherein said third detector includes an arm (81) which is movable between a first position in which said arm contacts with said processor connector and a second position in which said arm is pushed away from said first position and comes in contact with said endoscope connector connected to said processor connector, a biasing member (82) for pushing said arm against said first position, and a sensor (83) for sensing movement of said arm from said first position.

5. The processor of claim 4, wherein said third detector is located close to a center portion of said processor connector.

6. The processor of claim 5, wherein start of removal of said endoscope connector is detected by said third detector before being detected by said first and said second detectors.

7. The processor of claim 1, wherein said contacts used for said power supply are disposed close to a center portion of each of said endoscope connector and processor connector.

8. An endoscope system (2) comprising:
an endoscope (10) having an image sensor (50);
a display device (14); and
the processor (12) according to any of claims 1 to 7, and adapted to output image data, transmitted from said endoscope, to said display device (14).

## Patentansprüche

1. Prozessor (12) für ein Endoskop (10), umfassend:
einen Prozessorverbinder (40) passend zu einem Endoskopverbinder (24) an dem Endoskop, um das Endoskop und den Prozessor mechanisch sowie elektrisch miteinander zu verbinden;
einen Stromversorgungsabschnitt (62) zum Zuführen von Strom über den Prozessorverbinder und den Endoskopverbinder zu einem Bildsensor (50) in den Endoskop;
einen Treiber (52) zum Treiben des Bildsensors, indem dem Bildsensor über den Prozessorverbinder und den Endoskopverbinder ein Treibersignal zugeführt wird;
einen Detektorabschnitt (46e, 46f, 80) zum Nachweisen des Beginns des Abnehmens des Endoskopverbinders von dem Prozessorverbinder in einem Zustand, in welchem mindestens ein Paar Kontakte (46b, 36) für die Stromversorgung von mehreren Kontakten (46, 36) an dem Prozessorverbinder und dem Endoskopverbinder elektrisch angeschlossen bleibt; und
eine Steuerung (60) zum Steuern des Treibers und des Stromversorgungsabschnitts, um das Zuführen des Treibersignals zunächst anzuhalten und dann die Zufuhr von Strom ansprechend auf den Nachweis des Beginns des Entfernens des Endoskopverbinders einzustellen,
**dadurch gekennzeichnet, dass**
sowohl der Prozessorverbinder (40) als auch der Endoskopverbinder (24) einen Flachverbinder aufweist mit Kontakten, die linear in einer Richtung etwa orthogonal zu einer Einführ- und Ausziehrichtung des Endoskopverbinders verlaufen, und der Detektorabschnitt gebildet wird durch einen ersten Detektor (46e) und einen zweiten Detektor (46f), von denen der erste Detektor an einem seitlichen Endbereich des Prozessorverbinders vorgesehen ist, und der zweite Detektor an einem Endbereich der anderen Seite des Prozessorverbinders vorgesehen ist.

2. Prozessor nach Anspruch 1, bei dem der erste Detektor ein erster Kontakt (46e) ist, der an dem seitlichen Endbereich vorgesehen ist, und der zweite Detektor ein zweiter Kontakt (46f) an dem Endbereich auf der anderen Seite ist, und der Detektorabschnitt den Beginn des Entfernens des Endoskopverbinders nachweist, wenn das Leiten zwischen mindestens einem von dem ersten und dem zweiten Kontakt und einem entsprechenden Kontakt des Endoskopverbinders aufhört.

3. Prozessor nach Anspruch 1, bei dem der Detektorabschnitt einen dritten Detektor (80) enthält, der den Beginn des Entfernens des Endoskopverbinders dadurch nachweist, dass er einen mechanischen Versatz des Endoskopverbinders erfasst.

4. Prozessor nach Anspruch 3, bei dem der dritte Detektor einen Arm (61) enthält, der bewegbar ist zwischen einer ersten Stellung, in der der Arm den Prozessorverbinder kontaktiert, und einer zweiten Stellung, in der der Arm aus der ersten Stellung weggedrückt wird und in Kontakt mit dem Endoskopverbinder gelangt, der an den Prozessorverbinder angeschlossen ist, und ein Vorspannelement (82) aufweist zum Drücken des Arms in die erste Stellung, ferner einen Sensor (83) zum Fühlen der Bewegung des Arms aus der ersten Stellung.

5. Prozessor nach Anspruch 4, bei dem der dritte Detektor sich in der Nähe des Mittelbereichs des Prozessorverbinders befindet.

6. Prozessor nach Anspruch 5, bei dem der Beginn des Entfernens des Endoskopverbinders von dem dritten Detektor nachgewiesen wird, bevor dies von dem ersten und dem zweiten Detektor nachgewiesen wird.

7. Prozessor nach Anspruch 1, bei dem die für die Stromversorgung verwendeten Kontakte sich in der Nähe des Mittelbereichs sowohl des Endoskopverbinders als auch des Prozessorverbinders befinden.

8. Endoskopsystem (2), umfassend:
ein Endoskop (10) mit einem Bildsensor (50);
eine Anzeigevorrichtung (14); und
den Prozessor (12) nach einem der Ansprüche 1 bis 7, ausgebildet zum Ausgeben von Bilddaten, welche aus dem Endoskop übertragen werden, an die Anzeigevorrichtung (14).

## Revendications

1. Processeur (12) destiné à un endoscope (10), comprenant :
un connecteur de processeur (40) destiné à s'adapter à un connecteur d'endoscope (24) prévu sur ledit endoscope de manière à raccorder sur les plans mécanique et électrique ledit endoscope et ledit processeur ;
une partie d'alimentation en énergie (62) destinée à alimenter en énergie, par le biais dudit connecteur de processeur et dudit connecteur d'endoscope, un capteur d'image (50) prévu dans ledit endoscope ;
un circuit d'excitation (52) destiné à exciter ledit capteur d'image en fournissant un signal d'excitation audit capteur d'image par le biais dudit connecteur de processeur et dudit connecteur d'endoscope ;
une section de détection (46e, 46f, 80) destinée à détecter le début de retrait dudit connecteur d'endoscope dudit connecteur de processeur dans un état où au moins une paire de contacts (46b, 36) pour l'alimentation en énergie, parmi une pluralité de contacts (46, 36) sur ledit connecteur de processeur et ledit connecteur d'endoscope, est maintenue connectée sur le plan électrique, et
un contrôleur (60) destiné à contrôler ledit circuit d'excitation et ladite partie d'alimentation en énergie afin d'arrêter tout d'abord la fourniture dudit signal d'excitation, puis d'arrêter la fourniture en énergie en réaction à la détection d'un début de retrait dudit connecteur d'endoscope,
**caractérisé en ce que** chacun dudit connecteur de processeur (40) et dudit connecteur d'endoscope (24) comprend un connecteur de type plat présentant lesdits contact alignés de manière linéaire dans une direction pratiquement orthogonale à une direction d'introduction et de retrait dudit connecteur d'endoscope, et ladite section de détection est constituée d'un premier détecteur (46e) et d'un deuxième détecteur (46f), et ledit premier détecteur est prévu au niveau d'une partie d'extrémité latérale dudit connecteur de processeur, et ledit deuxième détecteur est prévu au niveau de l'autre partie d'extrémité latérale dudit connecteur de processeur.

2. Processeur selon la revendication 1, dans lequel ledit premier détecteur est un premier contact (46e) prévu au niveau de ladite partie d'extrémité latérale, et ledit deuxième détecteur est un second contact (46f) prévu au niveau de ladite autre partie d'extrémité latérale, et ladite section de détection détecte un début de retrait dudit connecteur d'endoscope lorsque la conduction entre au moins un desdits premier et second contacts et le contact correspondant dudit connecteur d'endoscope est interrompue.

3. Processeur selon la revendication 1, dans lequel ladite section de détection inclut un troisième détecteur (80) qui détecte le début de retrait dudit connecteur d'endoscope en détectant un déplacement mécanique dudit connecteur d'endoscope.

4. Processeur selon la revendication 3, dans lequel ledit troisième détecteur inclut un bras (81) pouvant être déplacé entre une première position, dans laquelle ledit bras est en contact avec ledit connecteur de processeur, et une seconde position, dans laquelle ledit bras est repoussé de ladite première position et vient en contact avec ledit connecteur d'endoscope connecté audit connecteur de processeur, un élément de sollicitation (82) destiné à pousser ledit bras contre ladite première position, et un capteur (83) destiné à détecter un déplacement dudit bras à partir de ladite première position.

5. Processeur selon la revendication 4, dans lequel ledit troisième détecteur se trouve à côté d'une position centrale dudit connecteur de processeur.

6. Processeur selon la revendication 5, dans lequel le début de retrait dudit connecteur d'endoscope est détecté par ledit troisième détecteur avant d'être détecté par lesdits premier et deuxième détecteurs.

7. Processeur selon la revendication 1, dans lequel lesdits contacts utilisés pour ladite alimentation en énergie sont disposés à proximité d'une partie centrale de chacun desdits connecteur d'endoscope et connecteur de processeur.

8. Système endoscopique (2), comprenant :
un endoscope (10) présentant un capteur d'image (50) ;
un dispositif d'affichage (14), et
le processeur (12) selon l'une quelconque des revendications 1 à 7, et apte à produire des données image, transmises à partir dudit endoscope, vers ledit dispositif d'affichage (14).
